# EUROPEAN PATENT APPLICATION

(11) **EP 2 700 654 A1**
(43) Date of publication of application: **26.02.2014**
(21) Application number: 12757506.6
(22) Date of filing: 19.03.2012
(51) Int. Cl.: C07K 17/08, C07K 1/107, A61P 3/10, A61K 38/28, C07K 14/62

(54) **HUMAN INSULIN AND ANALOG CONJUGATE THEREOF**

(30) Priority: 17.03.2011 CN 201110063821
(71) Applicant: Chongqing Fagen Biomedical Inc., Chongqing 400041 (CN)
(72) Inventor: FAN, Kai, Jiulongpo District Chongqing 400041 (CN); CAO, Yu, Jiulongpo District Chongqing 400041 (CN); CHEN, Yong, Jiulongpo District Chongqing 400041 (CN); LI, Hongliang, Jiulongpo District Chongqing 400041 (CN); CHEN, Hairong, Jiulongpo District Chongqing 400041 (CN); WAN, Ying, Jiulongpo District Chongqing 400041 (CN)
(74) Representative: Grünecker, Kinkeldey, Stockmair & Schwanhäusser
(86) International application number: PCT/CN2012/000346
(87) International publication number: WO 2012/122860

(57) **Abstract**

Disclosed is a recombinant human insulin or analog conjugate thereof. Said conjugate is formed by connecting the ε-amino group of lysine 29 of the B chain to activated polyethylene glycol by means of an amide bond. Further disclosed is a method of preparing said conjugate, a pharmaceutical compound, and a use for said conjugate in preparing medication for treating Type 1 and Type 2 diabetes.

Sequence Listing

<110>  CHONGQUING FAGEN BIOMEDICAL INC.

<120>  HUMAN INSULIN AND ANALOG CONJUGATE THEREOF


<130>  WP11-1686-XC66
<160>  3
<170>  PatentIn version 3.3


<210>  1
<211>  51
<212>  PRT
<213>  Homo sapiens

<400>  1
Gly Ile Val Glu Gln Cys Cys Thr Ser Ile Cys Ser Leu Tyr Gln Leu
1               5                   10                  15
Glu Asn Tyr Cys Asn Phe Val Asn Gln His Leu Cys Gly Ser His Leu
            20                  25                  30
Val Glu Ala Leu Tyr Leu Val Cys Gly Glu Arg Gly Phe Phe Tyr Thr
        35                  40                  45
Pro Lys Thr
    50


<210>  2
<211>  50
<212>  PRT
<213>  Artificial sequence

<400>  2
Gly Ile Val Glu Gln Cys Cys Thr Ser Ile Cys Ser Leu Tyr Gln Leu
1               5                   10                  15
Glu Asn Tyr Cys Asn Phe Val Asn Gln His Leu Cys Gly Ser His Leu
            20                  25                  30
Val Glu Ala Leu Tyr Leu Val Cys Gly Glu Arg Gly Phe Phe Tyr Thr
        35                  40                  45
Pro Lys
    50


<210>  3
<211>  197
<212>  DNA
<213>  Artificial sequence

<400>  3
ctcgagaaga gagaagtctt caagtttgtt aaccaacatt tgtgtggttc ccacttggtt      60

gaagctttgt acttggtttg tggtgaaaga ggtttcttct acactccaaa ggctgctaag     120

ggtatcgttg aacaatgttg tacttccatc tgttccttgt accaattgga aaactactgt     180

aactgataag cggccgc                                                    197

## Description

### Technical field

The present disclosure relates to biotechnology. More specifically, relates to recombinant preparation, modification, purification, and use of human insulin and analog thereof.

This application claims priority from the Chinese Patent Application No. 201110063821.6, filed March 17, 2011, the disclosure of which is hereby incorporated by reference herein in its entirety.

### Background

Insulin is a protein secreted by pancreatic β-cells under stimulation from endogenous or exogenous materials (such as glucose, lactose, ribose, arginine, and glucagon, *etc.*), and comprises 51 amino acids with a molecular weight of about 5,800 Dalton. An insulin molecule consists of an A-chain (21 amino acids) and a B-chain (30 amino acids), which are linked together by disulfide bond. And, if the disulfide bond is broken, the insulin will lose activity. A larger molecule, preproinsulin, is first synthesized by β-cell, and then processed into an 86-peptide proinsulin, which is further hydrolyzed into insulin and a linker. The main physiological effect of insulin is to regulate metabolic processes of: 1. promoting the intake and utilization of glucose in histocytes, promoting glycogen synthesis, inhibiting gluconeogenesis, and lowering blood sugar; 2. improving synthesis and storage of fatty acids, and decreasing lipolysis; and 3. facilitating the delivery of amino acids into cells, and enhancing synthesis rate of proteins.

Diabetes mellitus (DM) is a metabolic disease from diseased pancreas, characterized by chronic hyperglycemia accompanied by metabolic disorders of sugar, fat and protein from defects in insulin secretion or activity, in which hyperglycemia exhibits as a main feature. There are mainly two types of diabetes, the Type 1 (IDDM), cause by absolute insulin deficiency; and the Type 2 (NIDDM), cause by relative insulin deficiency. Diabetes has become a common and frequently-occurring disease, as well as the third disease after cancer and heart and cerebral vusculer diseases that threaten people's lives.

Insulin, one of the most effective medicaments for the treatment of diabetes, may regulate blood sugar at normal level via interreaction *in vivo* with insulin receptor on cytomembrane. Insulin receptor is a tetramer transmembrane glycoprotein, consisting of two α subunits and two β subunits that are linked by disulfide bond. The two α subunits, with insulin binding sites thereon, are located outside of plasma membrane; and the two β subunits are transmembrane proteins with signal transduction effect. With no insulin linked, the tyrosine protein kinase of the receptor exhibits no activity, and while the insulin binds to the α subunit of the receptor and changes the structure of the β subunit, the tyrosine protein kinase is activated and catalyses two reactions of: 1. phosphorylation of specific tyrosine residue on the β subunit of the tetramer complex, *i.e*., autophosphorylation; and, 2. binding and activating a second messenger by phosphorylated tyrosine residue IRSs, and amplifying and cascade signaling to conduct metabolic regulation. The degree of the insulin-receptor binding depends on amount of receptor as well as the affinity, which are both regulated by plasma insulin concentration. With the increase of insulin concentration, the amount of insulin receptor will decrease, *i.e*., be regulated down. Because of the decrease of receptor at fat cell membrane, obese patients suffered from non-insulin dependent diabetes exhibit insensitivity towards insulin, namely insulin resistance. By losing weight via diet control and physical exercises, receptors on fat cell membrane may be increased, and affinity thereof with insulin be enhanced, hence improving blood sugar utilization and regulating it back to normal level.

The higher the binding rate is between insulin and receptors, the more obvious the hypoglycemic activity is. Competitive binding test is performed for the assay of binding rate, wherein, receptors are incubated together with I¹²⁵-insulin with or without the addition of saturated and unlabeled insulin (or insulin analog). Radiolabeled and unlabeled insulin together result in total insulin binding, and radiolabeled I¹²⁵-insulin alone results in non-specific insulin binding. The specific insulin binding, obtained from the total minus the non-specific insulin binding, is divided by the total insulin concentration of the reaction system to generate specific binding rate. The receptor in competitive binding test is preferably rat liver insulin receptor purified by wheat germ agglutinin (WGA) agarose gel chromatography, because insulin receptor content is high in rat liver and thus facilitates the extraction, and meanwhile, the insulin-like growth factor-1 receptor, which is highly homologous with insulin receptor, expresses in a low level in liver and makes little interference towards the assay (D. R. Marshak, Strategies for Protein Purification and Characterization: A Laboratory Course Manual (in Chinese), Science Press, 1999, P141-192; Cheng Ningli et al., Functions of Hepatic Insulin Receptors in Neonatal Calves, Acta Universitatis Medicinalis Secondae Shanghai, 1997, 12(2): 23-25; Sinha MK., Subunit structure, autophosphorylation, and tyrosine-specific protein kinase activity of hepatic insulin receptors in fetal, neonatal, and adult rats., Diabetes, 1987, 36(1): 1161).

The physiological secretion of insulin in normal human body is regulated by blood sugar concentration. Insulin concentration reaches to peak after 30-60 min of a meal, and returns to normal after 2-4 hours, securing that blood sugar concentration does not change greatly with meals. After subcutaneous injection of natural human insulin to diabetic patients, the insulin diffuses around the injection site and plays a role in body circulation with, however, a very short effective acting time. Also, the frequent injection of insulin will lead inconveniences and sufferings to patients. Therefore, insulin with long effective acting time is required in clinical. Chemical modification is one of the approaches to obtain long-acting insulins. With chemical modification, insulin may exhibit longer half-life and lower antigenicity while remaining bioactivities. Chemical modification agents need to be stable, non-toxic, non-antigenic and of appropriate molecular weight. Polyethylene glycol (PEG) is a macromolecular compound with good biocompatibility and no toxicity to human bodies. To be conjugated with a target protein, PEG needs to be activated at its one end or two, and functional group is selected on the basis of characteristics of the molecule to be conjugated. Common biocompatible linking groups include ester, amido, imido, urethane, succinimidyl (e.g., succinimidyl succinate (SS), succinimidyl propionate (SPA), succinimidyl carboxymethyl (SCM) or N-hydroxyl-succinimidyl (NHS)), epoxyl, cysteinyl, histidinyl or primary amine. The activated PEG may theoretically be reacted with main amino acids (*e.g.,* Lys, Cys, His, Asp, Glu, Ser, and Thr, *etc.*) in a protein at the N- or C-terminus, in which a reaction with Lys at the N-terminus is the most common. There are three free amino groups on the molecule of recombinant human insulin or analog thereof, namely, the α-amino groups of GlyA¹ (pKa≈8.0) and PheB¹ (pKa<7.0), and the ε-amino group of LysB²⁹ (pKa≈9.5), which may all be employed for the chemical modification of insulin. Because the B²⁹ site of human insulin is not involved in receptor binding, covalent modifications thereon will not affect protein bioactivities (Murray-Rust J et al., BioEssays, 1992, 14 (5): 325-331). With pH>9.5, modifications at the ε-amino group of LysB²⁹ will be predominant with a high modification ratio (Hinds K et al., Bioconjug Chem, 2000, 11 (2): 195-201). Yu Zhengyan et al. (CN200410089050.8) disclose an mPEG-insulin complex, in which insulin and mPEG are conjugated via a free amino group of the former and an activated propionaldehyde group of the latter, and the molecular weight is 10.8-25.8 kD. However, the modification process is very complicated and the product is a mixture. Yin Chunhua et al. (CN200610118923.2) disclose a mono-modified PEGylated insulin (PheB1-PEG-insulin), wherein PEG is linked to the first Phe of the B chain of insulin via C-N bond, with a molecular weight of 6.55∼10.8 kD. However, the insulin acts very short *in vivo* with little hypoglycemic activity. In CN 1964899A, disclosed is a long-acting insulin derivative with maleoyl-PEG modification at site B29, said insulin binding to albumin after administration *in vivo.* In CN10721712A, disclosed is a method of double-chain PEGylation of insulin B30 complex. In CN101573173A, disclosed is a PEGylated prolonged insulin, with main PEGylation at the C-terminal carboxyl group of A21 and/or B30, as well as the N-terminal amino group of A1 and/or B1. In CN101045166A, disclosed is pulmonary administration of chemically modified insulin, wherein the chemical modification includes PEGylation on at least one site of A1, B1 or B29. In CN101743252A, disclosed is a PEGylated insulin analog that is stable to protease. The insulin analog comprises a structure different from human insulin, namely, comprising at least two hydrophobic amino acids substituted by hydrophilic ones. The analog is PEGylated at Lys B29 for pulmonary administration. In CN 200810232828.4, disclosed is a process for insulin PEGylation at A1 site only or at the two sites of A1 and B29. However, the insulin exhibits short-term and unstable activity of regulating blood sugar. The long-acting insulin detemir of Novo Nordisk is formed by conjugating myristic fatty acid at Lys29 of the B chain of insulin analog (see, CN101784563A, CN101389650A and EP2008/060734), exhibiting good effect of regulating blood sugar *in vivo* with 24-hour activity in human body and, however, only 12-house activity in animals.

In the invention, PEG is selected as a modification agent to be conjugated to the single site of the ε-amino group of lysine B29 of human insulin or DcsB30 recombinant human insulin via amide bond. After purification process, a conjugate with single modification site and stable activity is obtained. The binding rate between conjugates with different molecular weights and human insulin receptor is up to 40-90%, and the conjugates have significantly prolonged half-life *in vivo,* namely 24 to 72 hours of hypoglycemic activity in experimental animals (rabbits and dogs). The conjugate of recombinant human insulin and analog thereof of the invention may be used as a novel and long-acting medicament or in a pharmaceutical composition for the treatment of Type 1 and Type 2 diabetes.

### Detailed Description

One aim of the invention is to provide a conjugate of recombinant human insulin or analog thereof (*e.g.,* DesB30 recombinant human insulin). Said conjugate is formed by connecting the ε-amino group of lysine 29 of the B chain (Lys B29) to activated polyethylene glycol by means of a covalent bond. Said conjugate is of high binding rate towards insulin receptors as well as sustained hypoglycemic activities *in vivo.*

Another aim of the invention is to provide a method for the preparation of said conjugate, comprising preparation of monomers of recombinant human insulin or analog thereof, PEG conjugation, and protein purification thereafter.

Another aim of the invention is to provide a pharmaceutical composition of the conjugate of recombinant human insulin or analog thereof (*e.g.,* conjugate of DesB30 recombinant human insulin) for the treatment of Type 1 and Type 2 diabetes. Said composition comprises effective dose of the conjugate of recombinant human insulin or analog thereof (*e.g.,* conjugate of DesB30 recombinant human insulin) as an effective constituent. Said composition may further comprise pharmaceutically acceptable vectors and excipients.

Yet another aim of the invention is to provide use of said conjugate in the preparation of medicaments for the treatment of Type 1 and Type 2 diabetes. Said conjugate has a specific modification site, and includes conjugates that are PEGylated with PEG molecular weights of 5kDa, 10kDa, and 20kDa. Said conjugate is of high binding rate towards insulin receptors, prolonged half-life *in vivo* as well as sustained hypoglycemic activities, and hence may be used as a medicament or in pharmaceutical compositions for the treatment of Type 1 and Type 2 diabetes.

Therefore, in the first aspect of the invention, provided is a conjugate of recombinant human insulin or analog thereof (*e.g.,* DesB30 recombinant human insulin conjugate). Said conjugate is formed by connecting the ε-amino group of lysine 29 of the B chain (Lys B29) of said recombinant human insulin or analog thereof (*e.g.,* DesB30 recombinant human insulin) to activated polyethylene glycol by means of an amide bond.

Wherein, the amino acid sequence of recombinant human insulin or analog thereof to be PEGylated is as follows. The amino acid sequence of said recombinant human insulin is (SEQ ID No: 1): Gly Ile Val Glu Gln Cys Cys Thr Ser Ile Cys Ser Leu Tyr Gln Leu Glu Asn Tyr Cys Asn Phe Val Asn Gln His Leu Cys Gly Ser His Leu Val Glu Ala Leu Tyr Leu Val Cys Gly Glu Arg Gly Phe Phe Tyr Thr Pro *Lys* Thr, wherein, amino acids 1-21 constitute the A chain, and amino acids 22-51 constitute the B chain of the recombinant human insulin. Disulfide bonds are respectively formed between amino acids 6 and 11, 7 and 28, as well as 20 and 40, and lysine 29 of the B chain is shown in italic.

In the invention, said recombinant human insulin analog is a derived protein with substitution, deletion or addition of one or several amino acids in the amino acid sequence defined by SEQ ID NO: 1, while maintaining activities of recombinant human insulin.

Preferably, the amino acid sequence of said recombinant human insulin analog (DesB30 recombinant human insulin) is obtained by deletion of threonine 30 of the B chain of SEQ ID NO: 1, which may be named as DesB30 recombinant human insulin, which has an amino acid sequence of (SEQ ID NO: 2): Gly Ile Val Glu Gln Cys Cys Thr Ser Ile Cys Ser Leu Tyr Gln Leu Glu Asn Tyr Cys Asn Phe Val Asn Gln His Leu Cys Gly Ser His Leu Val Glu Ala Leu Tyr Leu Val Cys Gly Glu Arg Gly Phe Phe Tyr Thr Pro *Lys.* Wherein, amino acids 1-21 constitute the A chain of the DesB30 recombinant human insulin, and amino acids 22-50 constitute the B chain of the DesB30 recombinant human insulin. Disulfide bonds are respectively formed between amino acids 6 and 11,7 and 28, as well as 20 and 40, and lysine 29 of the B chain is shown in italic.

Said recombinant human insulin or analog thereof (*e.g.,* DesB30 recombinant human insulin) is prepared by genetic engineering technology. More specifically, a recombinant comprising the gene of recombinant human proinsulin precursor is constructed with a yeast expression vector and transformed into yeast. Expressor is selected and cultured in a fermentation tank for the expression of said recombinant human proinsulin precursor. The precursor protein is separated, purified, digested with trypsin to cleave leading peptide and short C-peptide, and separated by chromatography to obtain the DesB30 recombinant human insulin. Said DesB30 recombinant human insulin is then subject to chemical condensation reaction at the C-terminus (Gurramkonda et al., Microbial Cell Factories 2010, 9: 31; US4916212), and separated and purified to obtain recombinant human insulin. As shown in examples, both of the prepared recombinant human insulin and DesB30 recombinant human insulin exhibit purity of more than 98.0%, insulin activity of 27.5 IU/mg, as well as completely and correctly paired three disulfide bonds. The recombinant human insulin and DesB30 recombinant human insulin prepared with yeast disclosed herein may further be crystallized to obtain crystalline insulin.

PEGylated amino sites on the recombinant human insulin may include the two N-terminal α-aminos (Ala (A1) and Phe (B1)) and the Lys (B29) ε-amino of the insulin molecule. However, in the present invention, the PEGylated site is limited to the ε-amino of Lys B29. The ε-amino of Lys B29 of recombinant human insulin or DesB30 recombinant human insulin is connected to activate polyethylene glycol (*e.g.,* activated mPEG) via a covalent bond, preferably via a secondary amino or an amide bond, and more preferably via an amide bond.

Wherein, while activating PEG, an active group is connected to one end of PEG, and a blocking group is connected to the other. Wherein, said active group includes, but is not limited to succinimides (e.g. succinimidyl succinate (SS), succinimidyl propionate (SPA), succinimidyl carboxymethyl (SCM) or N-hydroxyl-succinimide (NHS)), nitrophenyl, amido, imido, carbamate, or aldehydyl group. Preferably, the linked active group is a succinimidyl propionate (SPA) group. The blocking group at the other end of PEG includes, but is not limited to methoxy, ethoxy, propoxy, butoxy, galactosyl or glucosyl, *etc.,* and preferably, the linked blocking group is a methoxy.

In the invention, said activated polyethylene glycol may be mPEG-succinimidyl propionate (mPEG-SPA), mPEG-succinimidyl esters (mPEG-NHS), mPEG-trichlorophenyl-carbonate (mPEG-TCP), or mPEG-aldehyde (mPEG-ALD). Preferably, said activated polyethylene glycol is mPEG-succinimidyl propionate (mPEG-SPA).

The activated polyethylene glycol may have branch chain or linear chain, preferably linear chain. The molecular weight of activated polyethylene glycol may be 5 kDa∼20 kDa. Preferably, the molecular weight of PEG may be 5kDa, 10kDa or 20kDa with mPEG-succinimidyl propionate (mPEG-SPA) as a conjugating molecule. Each of the recombinant human insulin or DesB30 recombinant human insulin of the invention couples with only one PEG molecule in said conjugate, without any multiple conjugation or non-B29 modifications.

According to the second aspect of the invention, provided is a method for the preparation of said conjugate of recombinant human insulin or analog thereof, including steps of coupling the ε-amino group of lysine B29 of said recombinant human insulin or analog thereof to activated polyethylene glycol via an amide bond, to form a conjugate.

Specifically, provided is a method for the preparation of said recombinant human insulin conjugate or DesB30 recombinant human insulin conjugate, including steps of:
a. expressing recombinant human insulin precursor with *Pichia pastoris,* trypsin-digesting to remove leading peptide and short C-peptide, and chromatographic-separating to obtain DcsB30 recombinant human insulin;
b. chemically condensation-reacting said DesB30 recombinant human insulin at the C-terminus and separating and purifying to obtain recombinant human insulin; and
c. coupling the ε-amino group of lysine B29 of said recombinant human insulin or the analog DesB30 recombinant human insulin to activated polyethylene glycol via an amide bond, to form the conjugate of said recombinant human insulin and the analog DesB30 recombinant human insulin of the invention. More specifically, mPEG-SPA (with molecular weight of 5kDa or 10kDa or 20kDa) is added twice into a solution of recombinant human insulin or DesB30 recombinant human insulin (20 mmol/L Tris-HCl, pH 10.5) with protein concentration of 2-4 mg/ml by protein-to-mPEG-SPA molar mass ratio of 1: 1.5-1: 2.5, followed by agitation at room temperature and 100 rpm for 20-40 min, and the reaction is terminated by adjusting pH to 3.0 by 1.0 mol/L of HCl. The product is then subject to reversed-phase column and cation column chromatography to remove non-modified insulin, PEG, and non-B29 conjugates, obtaining conjugate of recombinant human insulin and DesB30 recombinant human insulin with a purity of more than 98.0%. The product obtained according to the method of the invention has a specific modification rate of more than 40%. Also, the product is homogeneous and stable, as well as easy access.

An assay on the competitive binding to insulin receptor is performed to the conjugate of recombinant human insulin or DesB30 recombinant human insulin that is prepared by the method provided herein, and both result in high binding rate. Type 1 diabetes models (IDDM) of rabbits, dogs and mice are subcutaneously injected with appropriate dosage of the conjugate of recombinant human insulin or DesB30 recombinant human insulin, and exhibit significant effect of sustained lowering of blood sugar, suggesting that said conjugate may be used as a medicament or in pharmaceutical compositions for the treatment of Type 1 and Type 2 diabetes. Wherein, the conjugate comprising 5kDa PEG retains binding rate of over 75% with insulin receptors and may continuously lower the blood sugar for 12.5 hours *in vivo;* the conjugate comprising 10kDa PEG retains binding rate of over 60% with insulin receptors and may continuously lower the blood sugar for 23.8 hours *in vivo;* and the conjugate comprising 20kDa PEG retains binding rate of over 40% with insulin receptors and may continuously lower the blood sugar for 46.5 hours *in vivo.*

According to the third aspect of the invention, provided is a pharmaceutical composition of the conjugate of recombinant human insulin or analog thereof (*e.g.,* DesB30 recombinant human insulin) for the treatment of Type 1 and Type 2 diabetes. Said pharmaceutical composition comprises effective dosage of the conjugate of recombinant human insulin or analog thereof (*e.g*.DesB30 recombinant human insulin conjugate) as an effective constituent, and may further comprise pharmaceutically acceptable vectors and excipients. With studies in examples and reference to clinical therapeutic dosages, the inventor finds that said conjugate has an optimum formula by 0.01-0.5 mg/kg. Administration approach for the conjugate of the invention is mainly subcutaneous or intravenous injection, and may also include pulmonary or oral administration by techniques known in the art. Except for effective dose of recombinant human insulin conjugate, a formula for injection delivery needs also certain pH ranges (between 3.0-8.0), appropriate solution buffers (including, but being not limited to phosphate, citrate, acetate, glycine, and histidine), as well as protective agents (including, but being not limited to amino acid, zinc ion, monosaccharides or polysaccharides) and preservatives (including, but being not limited to phenol and m-cresol). A composition may be prepared by combining the human insulin conjugate of the invention with protamine or other human insulins (including, but being not limited to insulins in the market, such as insulin aspart, insulin lispro, regular insulin, insulin detemir, and insulin glargine) in a certain concentration.

According to the fourth aspect of the invention, provided is use of said conjugate of recombinant human insulin or analog thereof (*e.g.,* DesB30 recombinant human insulin conjugate) of the invention on the preparation of medicaments for the treatment of Type 1 and Type 2 diabetes. Said conjugate has a specific modification site and includes conjugates with PEGs with molecular weights of 5kDa, 10kDa, and 20kDa. Said conjugate is of high binding rate towards insulin receptors, prolonged half-life *in vivo* as well as sustained hypoglycemic activities.

The invention is described in details hitherto, and can be further illustrated with reference to the following examples, which are not meant to limit the present invention.

### Brief Description Of The Drawings

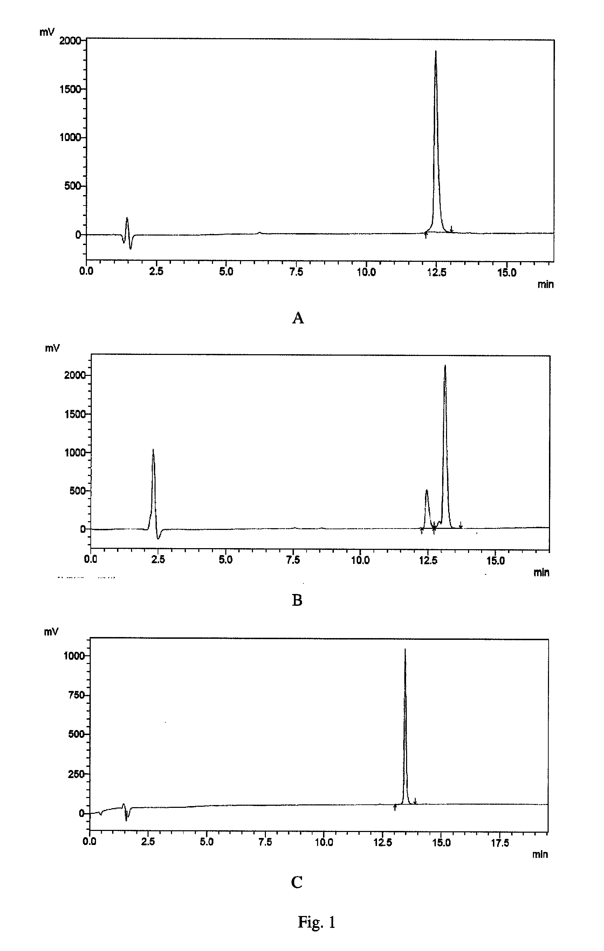

Fig. 1 shows HPLC results of DesB30 recombinant human insulin precursor and DesB30 recombinant human insulin, wherein, A is recombinant human DesB30 insulin precursor; B is DesB30 recombinant human insulin precursor digested by trypsin; and C is DesB30 recombinant human insulin.

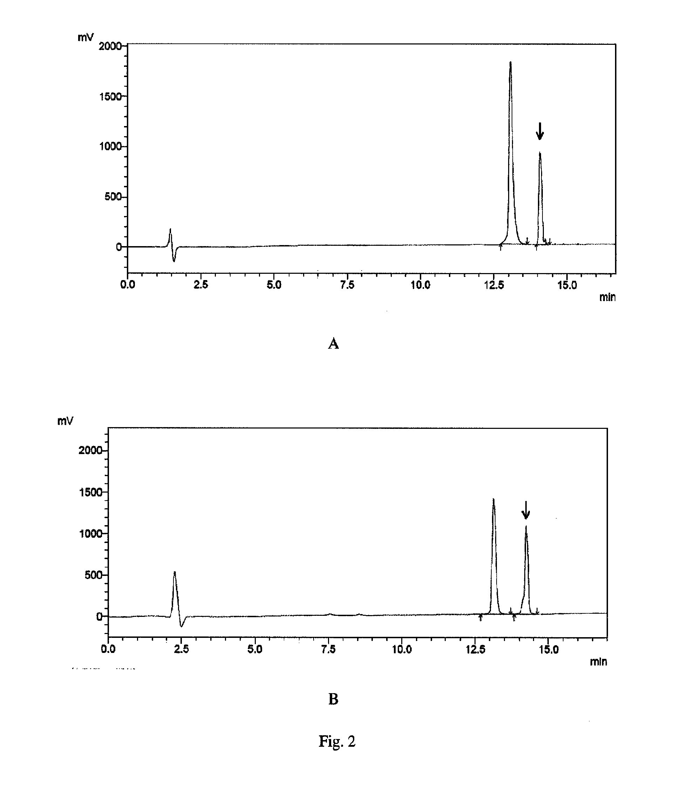

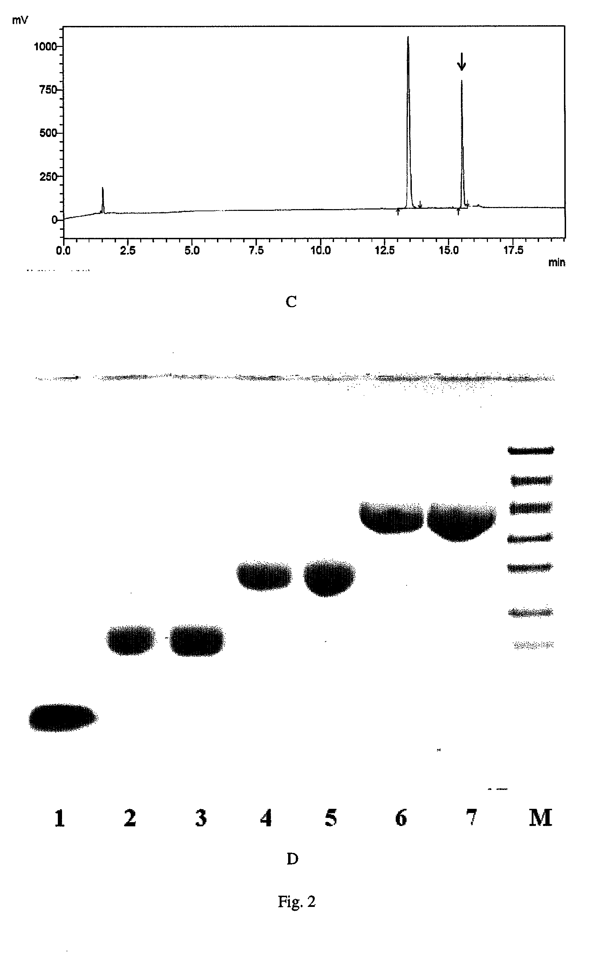

Fig. 2A shows C18 chromatography of DesB30-5K, wherein the arrow indicates the peak of collected B29-specific modification product. Fig. 2B shows C18 chromatography of DesB30-10K, wherein the arrow indicates the peak of collected B29-specific modification product. Fig. 2C shows C18 chromatography of DesB30-20K, wherein the arrow indicates the peak of collected B29-specific modification product. And Fig. 2D shows electrophotogram of DesB30 recombinant human insulin and conjugates of DesB30 recombinant human insulin with different molecular weights, wherein, 1 is non-modified DesB30; 2 is DesB30-5K; 3 is DesB30-5K; 4 is DesB30-10K; 5 is DesB30-10K; 6 is DesB30-20K; 7 is DesB30-20K; and M is a protein molecular weight standard, which represents, from bottom to top, 14.4 kD, 20 kD, 26 kD, 33 kD, 45 kD, 66.2 kD, and 94 kD.

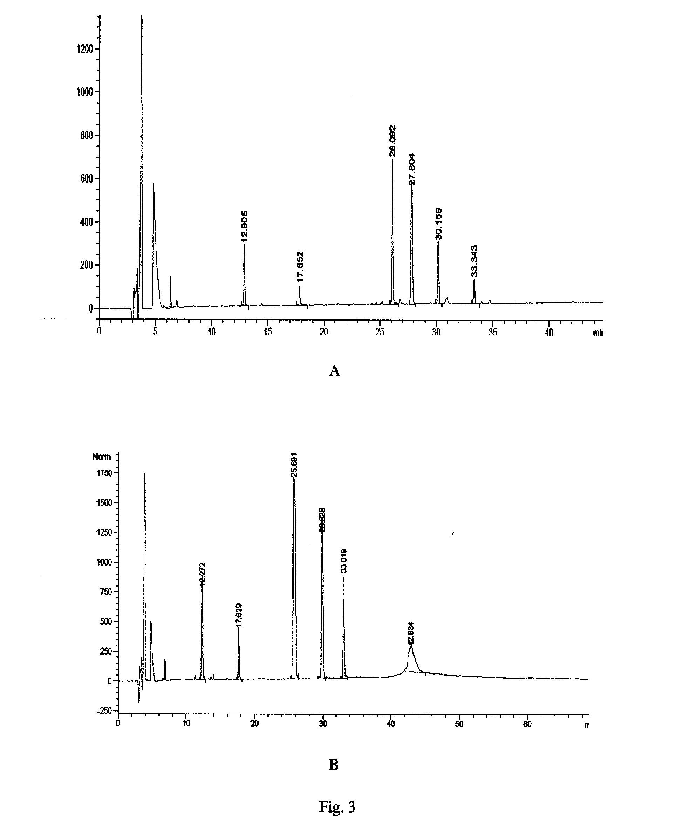

Fig. 3 shows reduced peptides of V8-protease digested DesB30 recombinant human insulin and recombinant human DesB30-20K, wherein, A is the reduced peptide of V8-protease digested DesB30 recombinant human insulin; and B is the reduced peptide of V8-protease digested recombinant human DesB30-20K.

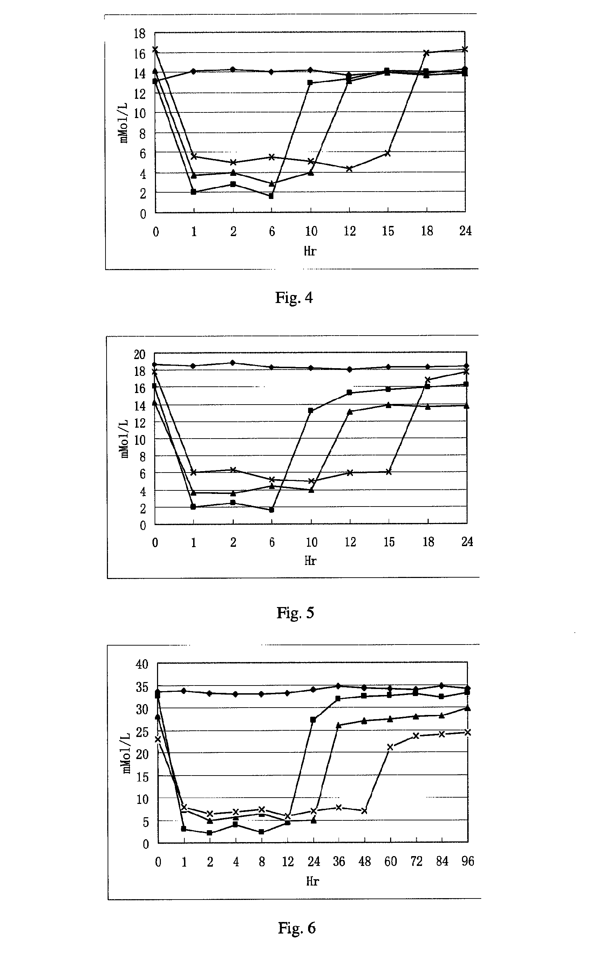

Fig. 4 shows time activity curve of DesB30 recombinant human insulin conjugate for the sustained regulation of blood sugar in Type 1 diabetes model of KunMing mice, wherein the X-axis represents the time (Hr) for the detection of empty stomach blood sugar in mice Type 1 diabetes model, and the Y-axis represents blood sugar value (mmol/L); wherein, ◆ is control group, ■ is DesB30-5K group, ▲ is DesB30-10K group, and × is DesB30-20K group.
Fig. 5 shows time activity curve of recombinant human insulin Ins conjugate for the sustained regulation of blood sugar in Type 1 diabetes model of KunMing mice, wherein the X-axis represents the time (Hr) for the detection of empty stomach blood sugar in mice Type 1 diabetes model, and the Y-axis represents blood sugar value (mMol/L); wherein, ◆ is control group, **■** is Ins-5K group, ▲ is Ins-10K group, and × is Ins-20K group.
Fig. 6 shows time activity curve of DesB30 recombinant human insulin conjugate for the sustained regulation of blood sugar in Type 1 diabetes model of New Zealand albino rabbits, wherein the X-axis represents the time (Hr) for the detection of empty stomach blood sugar in Type 1 diabetes model rabbits, and the Y-axis represents blood sugar value (mMol/L); wherein, ◆ is control group, ■ is DesB30-5K group, ▲ is DesB30-10K group, and × is DesB30-20K group.

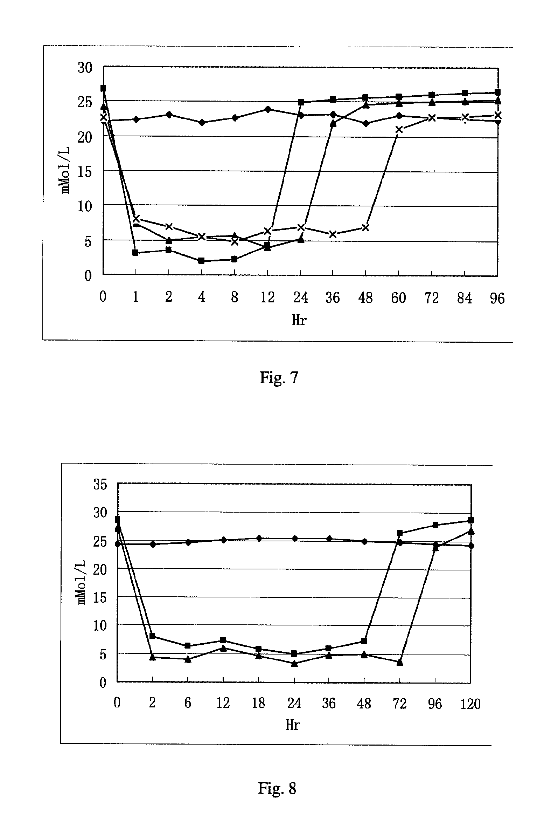

Fig. 7 shows time activity curve of recombinant human insulin (Ins) conjugate for the sustained regulation of blood sugar in Type 1 diabetes model of New Zealand albino rabbits, wherein the X-axis represents the time (Hr) for the detection of empty stomach blood sugar in Type 1 diabetes model rabbits, and the Y-axis represents blood sugar value (mMol/L); wherein, ◆ is control group, ■ is Ins-5K group, ▲ is Ins-10K group, and × is Ins-20K group.
Fig. 8 shows time activity curve of DesB30-20k conjugate with dosage gradient for the sustained regulation of blood sugar in Type 1 diabetes model of Beagle dogs (single administration), wherein the X-axis represents the time (Hr) for the detection of empty stomach blood sugar in the Type 1 diabetes model dogs, and Y-axis represents blood sugar value (mMol/L), wherein, ◆ is control group, ■ is dosage 0,05mg/kg group, and ▲ is dosage 0.1mg/kg group.

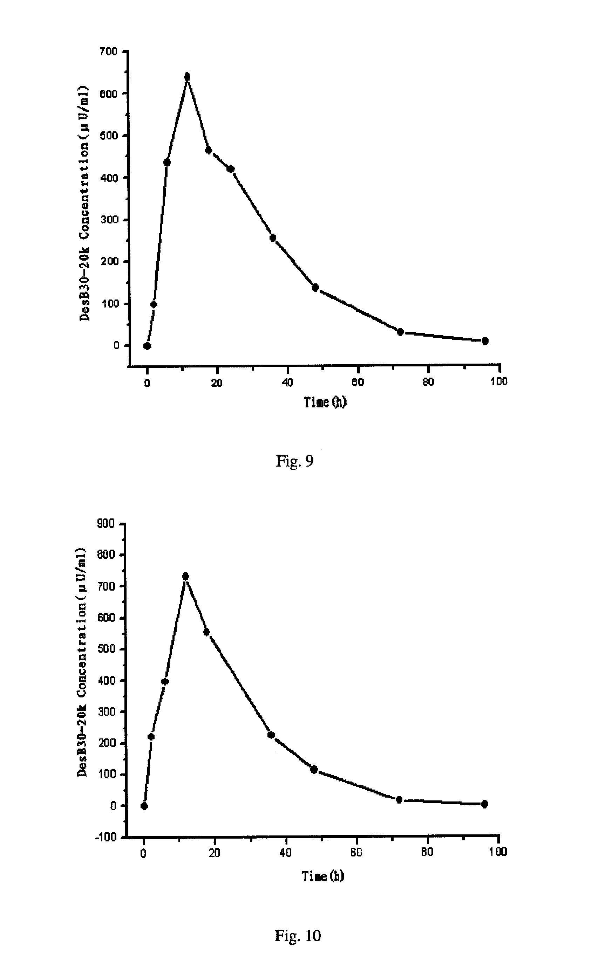

Fig. 9 shows blood drug levels in Type 1 diabetes model Beagle dogs that are subcutaneously injected with DesB30-20k conjugate for one time by 0.1mg/kg, wherein X-axis represents detection time (Hr), and Y-axis represents DesB30-20k conjugate concentration (µU/ml).
Fig. 10 shows blood drug levels in Type 1 diabetes model Beagle dogs that are subcutaneously injected with DesB30-20k conjugate for 6 times by 0.1mg/kg, wherein X-axis represents detection time (Hr), and Y-axis represents DesB30-20k conjugate concentration (µU/ml).

### Embodiments

The disclosure may be further understood by the following examples, which are not meant to be limitations. The recombinant human insulin (Ins) and analog thereof (DesB30) involved in the following examples are obtained via genetic engineering technology, with amino acid sequences respectively shown in SEQ ID NO: 1 and SEQ ID NO: 2 (SEQ ID No.1: Gly Ile Val Glu Gln Cys Cys Thr Ser Ile Cys Ser Leu Tyr Gln Leu Glu Asn Tyr Cys Asn Phe Val Asn Gln His Leu Cys Gly Ser His Leu Val Glu Ala Leu Tyr Leu Val Cys Gly Glu Arg Gly Phe Phe Tyr Thr Pro Lys Thr; SEQ ID No.2: Gly Ile Val Glu Gln Cys Cys Thr Ser Ile Cys Ser Leu Tyr Gln Leu Glu Asn Tyr Cys Asn Phe Val Asn Gln His Leu Cys Gly Ser His Leu Val Glu Ala Leu Tyr Leu Val Cys Gly Glu Arg Gly Phe Phe Tyr Thr Pro Lys; *see,* Gurramkonda C, Polez S, Skoko N, Adnan A, Gabel T, Chugh D, Swaminathan S, Khanna N, Tisminetzky S, and Rinas U., Application of simple fed-batch technique to high-level secretory production of insulin precursor using Pichia pastoris with subsequent purification and conversion to human insulin., Microb Cell Fact, May 12, 2010; 9 (1): 31).

### Example 1 Recombinant expression and purification of recombinant human insulin analog (DesB30)

In this example, cDNA sequence was designed on the basis of the amino acid sequence of human insulin analog DesB30 (SEQ ID NO: 2) with reference to code preference of yeast, said sequence comprising cDNAs corresponding to a leading peptide of EVFK, a middle short C-peptide AAK, as well as restriction sites of XhoI and NotI at the 5' and 3' terminus, respectively. The entire cDNA sequence is as followings (SEQ ID NO: 3): 5'-CTCGAGAAGAGAGAAGTCTTCAAGTTTGTTAACCAACATTTGTGTGGTTCCCACT TGGTTGAAGCTTTGTACTTGGTTTGTGGTGA AAGAGGTTTCTTCTACACTCCAAAGG CTGCTAAGGGTATCGTTGAACAATGTTGTACTTCCATCTGTTCCTTGTACCAATTGGA AAACTACTGTAACTGATAAGCGGCCGC-3'. The cDNA fragment of SEQ ID NO: 3 was artificially synthesized by TaKaRa Biotechnology (DaLian) Co., Ltd., and introduced into pUC18 vector (purchased from Invitrogen), the product being named as pUC18-DesB30. The plasmid pUC18-DesB30 and an expression vector plasmid pICZαA (purchased from Invitrogen) were double-digested with restriction enzyme Xho I (purchased from TaKaRa Biotechnology (DaLian) Co., Ltd.) and Not I (purchased from TaKaRa Biotechnology (DaLian) Co., Ltd.) and recovered from gels, then subject to T4 enzyme (purchased from TaKaRa Biotechnology (DaLian) Co., Ltd.) ligation, to construct and select an expression vector pICZαA-DesB30 comprising the DesB30 precursor gene. The recombinant plasmid was linearized with Avr II (purchased from TaKaRa Biotechnology (DaLian) Co., Ltd.) and electrotransformed into GS115 *Pichia pastoris* (purchased from Invitrogen). Colonies with high copy number were picked after His+ and Zeocin (1000 mg/ml) screen, innoculated into a 5 mL YPD medium, cultured overnight at 30°C, and transferred into a 250 mL YPD medium and cultured for 96 hours. Colonies with high expression were selected for the preparation of seed solutions, which were stored at -80°C in liquid nitrogen.

Fermentation and expression: seed solution was plated on YPD containing glucose 20 g/L, peptone 20 g/L, yeast extract 10 g/L, and agar 20 g/L, and cultured at 30 °C for 3-4 days. Colonies were picked from the YPD plate and innoculated into a 250 mL flask containing 25 mL YPD medium (containing glucose 20 g/L, peptone 20 g/L, and yeast extract 10 g/L), and cultured at 30 °C and 250 rpm for 24 hours to obtain Seed Liquid 1. The Seed Liquid 1 was innoculated by 1% into a 1000 mL flask containing 250 mL YPD medium, and cultured at 30 °C and 250 rpm over night to obtain Seed Liquid 2. The Seed Liquid 2 was innoculated by 10% into fermentation medium BSM (which contains, in each 1000 ml., H₃PO₄ (85%) 26.7 mL, CaSO₄·2H₂O 0.93 g, MgSO₄·7H₂O 14.9 g, KOH 4.13 g, K₂SO4 18.2 g, glycerol 40 g, microelements (PTM1) 4.4 mL and anti-foam 0.5mL; wherein the PTM1 contains, in each 1000 ml, CuSO₄·5H₂O 6 g, KCl 0.08 g, MnSO₄ 2.68 g, H₃BO₄ 0.029 g, Na₂MoO₄·2H₂O 0.2 g, ZnSO₄·7H₂O 20 g, FeSO₄·7H₂O 65 g, CoCl₂·6H₂O 0.916 g, H₂SO₄ 5 mL, and Biotin 0.2 g), and cultured at temperature of 25°C-30°C, pH 5.0 (adjusted with 28% ammonia water), initial agitation speed of 400rpm, initial air flow (filtrated air) of 10 L/min, and dissolved oxygen of about 96%. At the initial phase of fermentation, dissolved oxygen was the lowered from the increased oxygen consumption. By regulating air flow and agitation speed, the dissolved oxygen level was maintained above 30%. After the exhaustion of glycerol (for about 20-24 h, depending on initial inoculum size and culture conditions), dissolved oxygen was elevated as well as pH, and glycerol containing 12 mL/L PTM1 was restrictively fed for 2-30 hours according to growth status; followed by restrictively feeding of methanol containing 12 mL/L PTM1 with varied speeds according to growth status. The final concentration of methanol was maintained at 0.5∼1.0% with monitoring of gas chromatography till the end of fermentation, wherein, the methanol induction expression was maintained for a period of 48∼96 hours at a temperature of 20°C-30°C, preferably 22 °C, and a pH of 4-6, regulated by ammonia water. After fermentatioin, supernatant was collected by 10000 rpm centrifugation, and stored at -20 °C.

Crude purification: Fermentation broth was centrifuged at 4°C and 12000 rpm for 10 min. The supernatant was collected, adjusted to pH 8.0, and then subject to purification by Ni-chelated chromatography column for the recovery of DesB30 precursor, wherein, the condition of balance solution was 20 mmol/L Tris-HCl and 300 mmol/L NaCl, pH 8.0, and the condition of eluent was 20 mmol/L Tris-HCl, 50 mmol/L iminazole, and 300 mmol/L NaCl, pH 8.0. The eluted target protein was diluted by three times with Solution A, and then subject to Source 30Q purification, wherein the mobile phase Solution A was 20 mmol/L Tris-HCl (pH8.0), and mobile phase Solution B was 20 mmol/L Tris-HCl and 1000 mmol/L NaCl (pH8.0), with a linear clution by 0-100% of Solution B. Eluting peaks containing DesB30 precursor were collected (Fig. 1A).

DesB30 precursor digestion: Trypsin (purchased from Novagen) was added to Source 30Q eluted protein by mass ratio of 1: 1000, and pH was adjusted to 7.5-8.0, followed by mild agitation at room temperature for 12 hours. The digestion efficiency was no less than 90% (Fig. 1B).

Refined purification: The digested sample was further purified with preparative reverse phase C18 column (Gehealthcare), wherein the mobile phase Solution A was 0.1% trifluoroacetic acid and 5% acetonitrile, and mobile phase Solution B was 0.1% trifluoroacetic acid and 95% acetonitrile, with a linear elution by 0-100% of Solution B. Eluting peaks containing human insulin analog DesB30 were collected. The purity of the resulting DesB30 recombinant human insulin was determined to be 98.1% by RP-HPLC (C18 column from Waters Corp., wherein the mobile phase Solution A was 0.1% trifluoroacetic acid and 5% acetonitrile, and mobile phase Solution B was 0.1% trifluoroacetic acid and 95% acetonitrile, with a flow rate of 1 ml/min and a linear elution by 0-100% of Solution B) (Fig. 1C). The molecular weight was determined to be 5,705.56 by MALDI-TOF (Applied Biosystem, Type 4800), same as the theoretical value.

### Example 2 Preparation of recombinant human insulin analog conjugate (DesB30-5K)

100ml of recombinant human insulin analog (DesB30) solution with concentration of 2 mg/ml (containing 20 mmol/L Tris-HCl) was adjusted to pH 10.5, agitated by 100 rpm at room temperature, and added with mPEG-SPA (with a molecular weight of 5K, purchased from JENKEM TECHNOLOGY CO., LTD, BEIJING) by molar mass ratio of 1:1.5 to react for 30 min, followed by the addition of another portion of mPEG-SPA, with same ratio as above, to react for another 30 min. The reaction was terminated by adjusting pH to 3.0 with 1% trifluoroacetic acid, and the product was subject to preparative reverse phase C18 column chromatography to remove non-modified DesB30, free PEG molecules, and other non-B29 modified conjugates (see Fig. 2A, wherein the arrow indicates the peak of B29 modified conjugate), wherein, Solution A was 0.1% trifluoroacetic acid and 5% acetonitrile, Solution B was 0.1% trifluoroacetic acid and 95% acetonitrile, and DesB30-5K conjugate was collected with a linear gradient elution, and then lyophilized to dry powder (Fig. 2D).

### Example 3 Preparation of recombinant human insulin analog conjugate (DesB30-10K)

100ml of recombinant human insulin analog (DesB30) solution with concentration of 2 mg/ml (containing 20 mmol/L Tris-HCl) was adjusted to pH 10.5, agitated by 100 rpm at room temperature, and added with mPEG-SPA (with a molecular weight of 10K, purchased from JENKEM TECHNOLOGY CO., LTD, BEIJING) by a molar mass ratio of 1:2 to react for 30 min, followed by the addition of another portion of mPEG-SPA, with same ratio as above, to react for another 30 min. The reaction was terminated by adjusting pH to 3.0 with 1% TFA, and the product was subject to preparative reverse phase C18 column chromatography to remove non-modified DesB30, free PEG molecules, and other non-B29 modified conjugates (see Fig. 2B, wherein the arrow indicates the peak of B29 modified conjugate), wherein, Solution A was 0.1% trifluoroacetic acid and 5% acetonitrile, Solution B was 0.1% trifluoroacetic acid and 95% acetonitrile, and DesB30-10K conjugate was collected with a linear gradient elution, and then lyophilized to dry powder (Fig. 2D).

### Example 4 Preparation of recombinant human insulin analog conjugate (DesB30-20K)

100ml of recombinant human insulin analog (DesB30) solution with concentration of 2 mg/ml (containing 20 mmol/L Tris-HCl) was adjusted to pH 10.5, agitated by 100 rpm at room temperature, and added with mPEG-SPA (with a molecular weight of 20K, purchased from JENKEM TECHNOLOGY CO., LTD, BEIJING) by a molar mass ratio of 1:2.5 to react for 30 min, followed by the addition of another portion of mPEG-SPA, with same ratio as above, to react for another 30 min. The reaction was terminated by adjusting pH to 3.0 with 1% TFA, and the product was subject to preparative reverse phase C18 column chromatography to remove non-modified DesB30, free PEG molecules, and other non-B29 modified conjugates (see Fig. 2C, wherein the arrow indicates the peak of B29 modified conjugate), wherein, Solution A was 0.1% trifluoroacetic acid and 5% acetonitrile, Solution B was 0.1% trifluoroacetic acid and 95% acetonitrile, and DesB30-20K conjugate was collected with a linear gradient elution, and then lyophilized to dry powder (Fig. 2D).

### Example 5 Preparation of recombinant human insulin

The reaction system consisted of 10 mmol/L of DesB30 insulin precursor (from Source 30Q crude purification in Example 1), 200 µmol/L of TPCK treated trypsin, 0.8 mol/L of Thr(tBu)-OtBu, 7.0 mmol/L of CaCl₂, 50% 1:1 (v/v) of dimethyl sulfoxide/ethanol and 26% water, with pH adjusted to 6.0 with acetic acid. The system was reacted at 12°C for 24 h, followed by the addition of acetone (acidified with isovolumetric concentrated hydrochloric acid) to precipitate the protein. The B30 Thr(tBu)-OtBu human insulin was separated with preparative reverse phase C18 column, followed by de-protection with trifluoroacetic acid (TFA) and separation with cation chromatography (SP Sepharose FF), wherein the balanced solution was 20 mmol/L NaAC (pH 3.0), and the eluent was 20 mmol/L Na₂HPO₄ (pH 9.5), to obtain recombinant human insulin with purity of above 98.0%. The molecular weight was determined to be 5,824.66 by MALDI-TOF, same as the theoretical value.

### Example 6 Preparation of recombinant human insulin conjugates (Ins-5K, Ins-10K, and Ins-20K)

The recombinant human insulin conjugate (Ins-5K) was prepared as in Example 2, obtaining Ins-5K conjugate with a purity of above 98.0%.

The recombinant human insulin conjugate (Ins-10K) was prepared as in Example 3, obtaining Ins-10K conjugate with a purity of above 98.0%.

The recombinant human insulin conjugate (Ins-20K) was prepared as in Example 4, obtaining Ins-20K conjugate with a purity of above 98.0%.

### Example 7 Modification site assay of recombinant human insulin conjugates

Aim: To prove that conjugates Ins-5K, Ins-10K, and Ins-20K, as well as DesB30-5K, DesB30-10K and DesB30-20K of the examples were modified only at the Lys B29 side chain amino of recombinant human insulin, peptide mass assay was performed with V8-protease, which specifically digests the C-terminal peptide bond of Glu residue. Modification site and the specificity thereof were determined with combined results of LC-MS assay and amino acid sequencing of digested peptides of non-modified insulin and modified insulin conjugate.

Method: Samples of DesB30 and DesB30-20K were lyophilized, de-salted, and dissolved to 2 mg/ml with 1% NH₄HCO₃. 0.9 ml of each sample was added with 50µL of V8-protease (mass ratio 1:50, Sigma) and reacted at 37°C for 24 hours, followed by the addition of 10µL of 0.4 M TCEP and reaction at 37°C for 30 min. Peptide mass assay was performed with RP-HPLC (Agilent 1100) LC-MS, and the retention time and molecular weight of each peak was recorded.

Analysis: From the comparison between reduced peptides of DesB30 (Fig. 3A) and DesB30-20K (Fig. 3B), it is indicated that the No. 6 peptide fragment in Table 1, *i.e*., RGFFYTPK, disappears from the reduced peptide of DesB30-20K, whereas the other 5 peptide fragments are the same in both reduced peptides. The peptide fragment of 42.834 min in reduced peptide of DesB30-20K is a PEGylated peptide (determined by evaporative light scattering), indicating that the PEGylation site is located on said fragment.

**Table 1**

| Nos. | Peptide fragments | Theoretical molecular weight (MW) | Retention time (min) | Determined molecular weight (MW) | SEQ ID NO: |
|---|---|---|---|---|---|
| 1 | GIVE | 416.23 | 17.852 | 415.30 | 4 |
| 2 | QCCTSICSLYQLE | 1489.63 | 33.343 | 1488.53 | 5 |
| 3 | NYCN | 512.17 | 112.905 | 511.20 | 6 |
| 4 | FVNQHLCGSHLVE | 1481.71 | 26.092 | 1480.74 | 7 |
| 5 | ALYLVCGE | 866.42 | 30.159 | 865.33 | 8 |
| 6 | RGFFYTPK | 859.4348 | 27.804 | 858.42 | 9 |

Then, the 42.834 min peptide was purified with semi- preparative reverse phase C18 column RP-HPLC (Gehealthcare), and sequenced (in Institute of Biochemistry and Cell Biology, SIBS, CAS, China) to be RGFFYTPX (SEQ ID NO: 10), wherein X is the amino acid being not detected, indicating that the Lys is PEGylated in said fragment. The above result indicates that the only modification site of DesB30-20K is amino acid B29. The other 5 conjugates (Ins-5K, Ins-10K, Ins-20K, DesB30-5K and DesB30-10K) were assayed by same means, resulting also the same.

### Example 8 Blood sugar lowering in mice Type 1 diabetes model

70 healthy KunMing male SPF mice (purchased from the Third Military Medical Univ., China) with body weight of 25-30 g were fed with water only and no food for 14 hours, and then subject to 120 mg/kg of rapid intraperitoneal injection of streptozotocin (STZ) solution (4.8mg/ml) that was prepared with citric acid- trisodium citrate buffer, to establish a Type 1 diabetes model. After the injection, the mice were immediately fed with food and water. After 48 hours of model establishing, blood was sampled from caudal vein, and blood sugar value was detected with glucometer (Bayer's Fast Glucometer) after fasting for 2 hours, wherein a blood sugar value of ≥11.1 mmol/L indicates successful modeling. 40 modeled mice were randomly divided into 4 groups, 10 for each group (group A, control, group B, DesB30-5K, group C, DesB30-10K, and group D, DesB30-20K). Groups B, C, and D were respectively subject to subcutaneous administration by a dosage of 0.25 mg/kg, and group A was injected with isovolumetric solvent. Before administration (*i.e*., 0 hour) and after 1, 2, 6, 10, 15, 18, and 24 hours of administration, blood was sampled from caudal vein for the detection of empty stomach (fasting for 2 hours) blood sugar value. The average blood sugar value was calculated and time activity curve was made (Fig. 4).

Results show that DesB30-5K, DesB30-10K, and DesB30-20K all exhibit significant hypoglycemic activity, wherein the lowering effect of DesB30-5K may sustain for about 8 hours, the lowering effect of DesB30-10K may sustain for about 12 hours, and the lowering effect of DesB30-20K may sustain for about 18 hours.

Experiment method for the detection of hypoglycemic activity of recombinant human insulin conjugates (Ins-5K, Ins-10K, Ins-20K) on KunMing mice Type 1. diabetes model was same as above. The average blood sugar value was calculated and time activity curve was made (Fig. 5). Results show that, the lowering effect of Ins-5K may sustain for about 8 hours, the lowering effect of Ins-10K may sustain for about 12 hours, and the lowering effect of Ins-20K may sustain for about 18 hours.

### Example 9 Blood sugar lowering experiment on Type 1 diabetes model (IDDM) rabbits

40 male New Zealand albino rabbits (purchased from the Third Military Medical Univ., China) with body weight of 2-3 kg were fed with water only and no food for 24 hours, and then subject to 5 ml/kg of ear vein single injection of tetraoxypyrimidine solution (with concentration of 20mg/ml) to establish models. After the injection, the rabbits were immediately fed with food and water. After 3 days of model establishing, blood was sampled every day from ear vein after fasting for 6 hours, and blood sugar value was detected with glucometer, wherein a blood sugar value of ≥11.1 mmol/L indicates successful modeling. 24 modeled rabbits were randomly divided into 4 groups, 6 for each group (group A, control, group B, DesB30-5K, group C, DesB30-10K, and group D, DesB30-20K). Groups B, C, and D were respectively subject to subcutaneous administration by a dosage of 0.2 mg/kg, and group A was injected with isovolumetric solvent. Before administration (*i.e*., 0 hour) and after 1, 2, 4, 8, 12, 24, 36, 48, 60, 72, 84, and 96 hours of administration, blood was sampled from ear vein for the detection of empty stomach (fasting for 6 hours) blood sugar value (Fast Glucometer, Bayer). The average blood sugar value was calculated and time activity curve was made (Fig. 6). Results show that DesB30-5K, DesB30-10K, and DesB30-20K all exhibit significant hypoglycemic activity on rabbit Type 1 diabetes model (IDDM), wherein the lowering effect of DesB30-5K may sustain for about 15 hours, the lowering effect of DesB30-10K may sustain for about 30 hours, and the lowering effect of DesB30-20K may sustain for about 55 hours.

Experiment method for the detection of hypoglycemic activity of recombinant human insulin conjugates (Ins-5K, Ins-10K, Ins-20K) on rabbit Type 1 diabetes model was same as above. The average blood sugar value was calculated and time activity curve was made (Fig. 7). Results show that, the lowering effect of Ins-5K may sustain for about 15 hours, the lowering effect of Ins-10K may sustain for about 35 hours, and the lowering effect of Ins-20K may sustain for about 55 hours.

### Example 10 Detection of binding rate towards insulin receptor

Rat liver insulin receptor extraction and receptor binding rate detection were performed with reference to literature (Sinha MK., Subunit structure, autophosphorylation, and tyrosine-specific protein kinase activity of hepatic insulin receptors in fetal, neonatal, and adult rats., Diabetes, 1987; 36 (1): 1161) as follows: 200 g of liver tissue was taken from 2-week old rat (purchased from the Laboratory Animal Center of the Third Military Medical Univ., China) at 4°C, homogenated after the addition of Buffer A (25 mmol/L of 4-hydroxyethyl piperazine ethanesulfonic acid (*i.e.*, Hepes), 0.25 mol/L of sucrose, 5 mmol/L of EDTA-Na, and 10 mmol/L of PMSF), and then subject to 2000xg centrifugation for 10 min. The resulting supernatant was centrifuged by 50000×g for 45min, and the precipitate was added with Buffer A, mixed, added by 10: 1 with a lysate solution (2mg/ml of Bacitracin, 2mmol/L of PMSF, and 2% Triton X-100), and then subject to 37°C incubation for 30 min and 120000×g centrifugation for 30min, and the supernatant was taken. A chromatography column was prepared with 3.0 ml of WGA-Sepharose, washed with a Washing Solution (containing 25 mm/L of Hepes, 0.05% Triton X-100, 100 mm/L of NaCl, 2.5 mmol/L of KCL, and 1.0 mmol/L of CaCl₂), and eluted with an Eluent (containing 0.3 mmol/L of N-acetyl glucosamine, and 10% glycerin). Rat liver insulin receptor protein concentration was detected by Lowry method and adjusted to 2.0 mg/ml. Reaction systems were then established in 7 micro centrifuge tubes, as follows (*see* Table 2), wherein I¹²⁵ insulin was purchased from the Atomic Energy Research Center of China, and the binding buffer contained 25 mmol/L of Hepes, 0.05% Triton X-100 and 0.1.% BSA. After reaction at 4°C for 16 hours, the system was added with 100 µl of 0.04% γ -globulin and 400 µl of 20% PEG (8000), mixed, and centrifuged by 12000xg for 30 min. The supernatant was removed, and intensity of radioactivity (cpm) in each tube was detected in a γ-scintillation counter. The specific-binding cpm value was obtained by taking the cpm value of tube 7 (the non-specific and background cpm value) from the cpm value of tubes 1-6, respectively. The proportion of receptor binding insulin equals to each specific-binding cpm value divided by the total cpm value, the concentration of ligand-receptor complex (HR) equals to the proportion multiplied by insulin concentration in each tube, and the free ligand concentration (H) equals to insulin concentration minus ligand-receptor complex. Finally, Scatchard analysis was performed to the HR and H, with the binding rate between recombinant human insulin Ins (recombinant human insulin standard preparation from the National Institutes for Food and Drug control, China, 20 mg for each) and the receptor being set as 100%, to calculate the binding rate between each recombinant insulin conjugate and the receptor.

**Table 2**

| Nos. | 2.0 mg/ml of insulin receptor protein (µl) | 5 nmol/L of I¹²⁵-insulin (µl) | 20 nmol/L of insulin conjugate (µl) | 600 nmol/L of insulin conjugate (µl) | Binding buffer (µl) | 1% BSA (µl) | Total insulin concentration (nmol/L) |
|---|---|---|---|---|---|---|---|
| 1 | 40 | 30 | 0 | 0 | 200 | 30 | 0.50 |
| 2 | 40 | 30 | 5 | 0 | 195 | 30 | 0.83 |
| 3 | 40 | 30 | 20 | 0 | 180 | 30 | 1.83 |
| 4 | 40 | 30 | 60 | 0 | 140 | 30 | 4.50 |
| 5 | 40 | 30 | 0 | 5 | 195 | 30 | 10.50 |
| 6 | 40 | 30 | 0 | 10 | 190 | 30 | 20.50 |
| 7 | 40 | 30 | 0 | 100 | 100 | 30 | 200.50 |

The calculated binding rate between each recombinant insulin conjugate and the receptor is shown in Table 3.

**Table 3**

| Conjugates | DesB30-5K | Ins-5K | DesB30-10K | Ins-10K | DesB30-20K | Ins-20K |
|---|---|---|---|---|---|---|
| Binding rates | 81.7% | 83.5% | 68.2% | 70.7% | 53.7% | 48.2% |

### Example 11 Blood sugar lowering experiment of DesB30-20k conjugate on Beagle dog Type 1 diabetes model

20 healthy Beagle dogs (purchased from the Third Military Medical Univ., China) with body weight of 5-8 kg were fed with water only and no food for 24 hours, and then subject to foreleg subcutaneous vein injection of tetraoxypyrimidine solution (200 mg/ml) and STZ solution (120mg/ml), with respective dosage of 50mg/kg and 30mg/kg, to establish Type 1 diabetes models. After the injection, the dogs were immediately fed with food and water. After 72 hours of model establishing, successful modeling was indicated by the signs of polyphagia, polydipsia, polyuria, lose weight, and blood sugar value of ≥11.1 mmol/L. 12 modeled dogs were randomly divided into 3 groups, 4 for each group (group A, control, group B, administration by 0.05 mg/kg, and group C, administration by 0.10 mg/kg), and subject to subcutaneous administration for one time. After 0, 2, 6, 12, 18, 24, 36, 48, 72, 96, and 120 hours of administration, blood was sampled from hindlimb vein for the detection of empty stomach (fasting for 6 hours) blood sugar value (GOD-POD method). The average blood sugar value was calculated and single administration time activity curve was made (Fig. 8). Results of single administration show that DesB30-20k may effectively lower blood sugar for 72 hours by 0.10 mg/kg. Then, the model animals were tested with multiple times of subcutaneous administration, with an interval of 72 hours (with a dosage of 0.05 mg/kg, 0.10 mg/kg, or isovolumetric solvent) for 6 times. From Day 1 to Day 21 after the administration, empty stomach blood sugar value was detected every three days, and blood was sampled after the last administration for pharmacokinetics experiments. Results show that, DesB30-20K exhibits significant hypoglycemic activity on Beagle dogs Type 1 diabetes model with a remarkable dose-effect relationship. The durations of hypoglycemic activity for single and multiple administrations are basicly the same, *i.e*., 72 hours. During the multiple administrations, the empty stomach blood sugar value of animals in administration groups were kept in average with about 5 mmol/L, whereas the empty stomach blood sugar value of animals in control group were kept in average with about 25 mmol/L.

### Example 12 Pharmacokinetics of the conjugate DesB30-20K of recombinant human insulin in dogs

After 0, 2, 6, 12, 18, 24, 36, 48, and 72 hours of the single administration and 0, 2, 6, 12, 18, 24, 36, 48, and 72 hours of the 6^{th} administration of the multiple administrations in Example 11, blood was respectively sampled and the average blood drug level of the 4 dogs was detected and calculated with Radio-immunity Kit for dog insulin (purchased from Beijing North Institute of Biological Technology), and pharmacokinetics parameters were calculated by DAS2.0 software, to make the blood drug level curve (Fig. 9 and Fig. 10).

Results show that, for single administration of DesB30-20K, the elimination half life (t_{1/2}) is 9.2 h, the area under curve (AUC) is 15,215.8 µIU/ml*h, and the peak concentration (Cmax) is 930.1 µIU/ml*h; for multiple administrations (with an interval of 72 hours, for 6 times), the elimination half life (t_{1/2}) is 9.4 h, the area under curve (AUC) is 15,391.6 µIU/ml*h, and the peak concentration (Cmax) is 953.2 µIU/ml*h. The pharmacokinetics result is in accordance with the blood sugar lowering potency experiment, indicating that DesB30-20K is characterized in long half life *in vivo,* and that, by continuous administration with an interval of 72 hours for 6 times, no DesB30-20K accumulation was found *in vivo.*

## Claims

1. A conjugate of recombinant human insulin or analog thereof, wherein said conjugate is formed by connecting the ε-amino group of lysine 29 of the B chain of said recombinant human insulin or analog thereof to an activated PEG by means of a covalent bond; preferably, said covalent bond is a secondary amine or an amide bond; and more preferably, said covalent bond is an amide bond.

2. Said conjugate of recombinant human insulin or analog thereof according to claim 1, wherein the amino acid sequence of said recombinant human insulin is shown in SEQ ID No: 1, and the analog of said recombinant human insulin is DesB30 recombinant human insulin, the amino acid sequence of which is shown in SEQ ID No: 2.

3. Said conjugate of recombinant human insulin or analog thereof according to claims 1 or 2, wherein said recombinant human insulin or analog thereof is conjugated only at the ε-amino group of lysine 29 of the B chain to the activated PEG.

4. Said conjugate of recombinant human insulin or analog thereof according to any one of claims 1∼3, wherein that an active group is connected to one end of said activated PEG, and a blocking group is connected to the other, wherein said active group includes succinimide, nitrophenyl, amido, imido, carbamate, or aldehydyl group, wherein, said succinimide includes succinimidyl succinate, succinimidyl propionate, succinimidyl carboxymethyl or N-hydroxyl-succinimide; and/or, wherein, said blocking group includes methoxy, ethoxy, propoxy, butoxy, galactosyl or glucosyl; and more perferably, wherein, said active group is succinimidyl succinate; and/or, said blocking group is methoxy.

5. Said conjugate of recombinant human insulin or analog thereof according to any one of claims 1∼4, wherein said activated PEG is mPEG-succinimidyl propionate, mPEG-succinimidyl esters, mPEG-trichlorophenyl-carbonate or mPEG-aldehyde.

6. Said conjugate of recombinant human insulin or analog thereof according to any one of claims 1∼5, wherein said activated PEG is mPEG-succinimidyl propionate.

7. Said conjugate of recombinant human insulin or analog thereof according to claim 1, wherein said activated PEG molecule has a branch or linear chain, and a molecular weight of 5 kDa∼20 kDa; preferably, said activated PEG has a linear chain, and a molecular weight of 5 kDa, 10 kDa or 20 kDa.

8. A method for the preparation of said conjugate of recombinant human insulin or analog thereof according to claim 1, including the step of linking the ε-amino group of lysine B29 of said recombinant human insulin or analog thereof to activated PEG via an amide bond to form a conjugate.

9. A pharmaceutical composition, wherein said composition comprises effective dose of said conjugate of recombinant human insulin or analog thereof of any one of claims 1∼7 as an effective constituent, and said composition may further comprise pharmaceutically acceptable vectors and excipients.

10. Use of said conjugate of recombinant human insulin or analog thereof of any one of claims 1∼7 in the preparation of medicaments for the treatment of Type 1. and Type 2 diabetes.
